(19) **Europäisches Patentamt / European Patent Office / Office européen des brevets**

(11) **EP 1 858 404 B1**

(12) # EUROPEAN PATENT SPECIFICATION

(45) Date of publication and mention
of the grant of the patent:
**14.04.2010 Bulletin 2010/15**

(21) Application number: **06727615.4**

(22) Date of filing: **09.02.2006**

(51) Int Cl.:
***A61B 5/026*** (2006.01)    ***G06F 17/00*** (2006.01)

(86) International application number:
**PCT/IB2006/050425**

(87) International publication number:
**WO 2006/085278 (17.08.2006 Gazette 2006/33)**

(54) **OPTICAL BLOOD FLOW SENSOR USING SELF-MIXING DOPPLER EFFECT**

OPTISCHER BLUTFLUSSMESSER UNTER VERWENDUNG VON SELBSTMISCH-DOPPLEREFFEKT

DETECTEUR DE DEBIT SANGUIN

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.02.2005 EP 05300108**

(43) Date of publication of application:
**28.11.2007 Bulletin 2007/48**

(73) Proprietor: **Koninklijke Philips Electronics N.V.**
**5621 BA Eindhoven (NL)**

(72) Inventors:
• **PRESURA, Cristian**
**F-75008 Paris (FR)**
• **AKKERMANS, Antonius**
**F-75008 Paris (FR)**
• **HEINKS, Carsten**
**F-75008 Paris (FR)**
• **DAMSTRA, Marijn**
**F-75008 Paris (FR)**
• **TACHE, Daniela**
**F-75008 Paris (FR)**

(74) Representative: **Schouten, Marcus Maria**
**Philips**
**Intellectual Property & Standards**
**P.O. Box 220**
**5600 AE Eindhoven (NL)**

(56) References cited:
• **OZDEMIR S K ET AL: "Noninvasive blood flow measurement using speckle signals from a self-mixing laser diode: in vitro and in vivo experiments" OPTICAL ENGINEERING SPIE USA, vol. 39, no. 9, September 2000 (2000-09), pages 2574-2580, XP002391510 ISSN: 0091-3286**
• **MEIGAS K ET AL: "Self-mixing in a diode laser as a method for cardiovascular diagnostics" JOURNAL OF BIOMEDICAL OPTICS SPIE USA, vol. 8, no. 1, January 2003 (2003-01), pages 152-160, XP002391511 ISSN: 1083-3668**
• **MEIGAS K ET AL: "Simple coherence method for blood flow detection" PROCEEDINGS OF THE SPIE - THE INTERNATIONAL SOCIETY FOR OPTICAL ENGINEERING SPIE-INT. SOC. OPT. ENG USA, vol. 3915, 2000, pages 112-120, XP002391512 ISSN: 0277-786X**
• **BONNER R ET AL: "Model for laser Doppler measurements of blood flow in tissue" APPLIED OPTICS USA, vol. 20, no. 12, 15 June 1981 (1981-06-15), pages 2097-2107, XP002391513 ISSN: 0003-6935**

# Description

The present invention relates to a method for measuring the blood flow in living tissue, and a device for performing the method.

Such a device and corresponding method is disclosed e.g. in EP 282210 A1. This device uses a laser and a linear light sensor. The light sensor registers light from the laser that is reflected by the skin of a user, and the user's pulse may be determined based on this signal.

A disadvantage with such a device is that its signal will mainly depend on the skin movement, and will therefore not measure the actual blood flow. This means that determining other blood-flow characteristics than pulse will be difficult.

It is therefore an object of the present invention to provide a blood flow sensing method that is capable of providing actual blood flow parameters in a reliable manner.

More specifically according to a first aspect, the invention relates to a method for measuring the blood flow in living tissue, comprising the following steps: illuminating the tissue with a laser beam, using a laser, and allowing a part of the laser beam, scattered by the tissue, to re-enter into the laser; measuring, using a photo sensor, light emitted from the laser, thus obtaining a signal which varies in accordance with the interference between the original laser beam and the scattered laser beam; applying a Fourier transform to said signal in order to provide the spectrum of the signal; and applying an exponential fit to said spectrum, thereby obtaining parameters corresponding to the average blood cell velocity in the tissue and the amount of blood in the tissue.

The use of such a sensor, that utilizes a self-mixing effect, capable of providing data of actual blood cell movements in the tissue, together with a Fourier analysis and use of an exponential model, allows reliable determination of actual blood flow parameters. These parameters may be used not only to determine a user's pulse, but also e.g. a measure of the perfusion in the tissue.

Use of self-mixing laser diodes in order to determine blood flow parameters is for example described in the following documents:

- "Non invasive blood flow measurement using speckle signals from a self-mixing laser diode: in vitro and in vivo experiments"; Optical Engineering SPIE USA; Vol. 39, n° 9; September 2000; P. 2574-2580;

- "Self-mixing in a diode laser as a method for cardiovascular diagnostics"; Journal of Biomedical Optics SPIE USA; Vol. 8, n° 1; January 2003; P. 152-160;

- "Simple coherence method for blood flow detection"; Proceedings of the SPIE - The International Society for Optical Engineering; Vol. 3915, 2000, P. 112-120.

In a preferred embodiment, the photo sensor signal is measured during a time period of 5-15 ms for each generated spectrum. This provides even more reliable parameters, since the amount of blood cells in the tissue, as well as the average velocity of these cells, can be regarded as constant during such a time period.

Preferably, a parameter set is collected from a number of such time periods. From such a parameter set, e.g. the user's pulse may be determined.

It is also possible to determine a perfusion parameter, by multiplying the parameter, corresponding to the average blood cell velocity in the tissue, and the parameter, corresponding to the amount of blood in the tissue.

In a preferred embodiment, the exponential fit is applied in an interval from 0.2 kHz to 10 kHz.

According to a second aspect, the invention relates to a device for measuring the blood flow in living tissue. The device comprises a laser for illuminating the tissue with a laser beam, the laser being adapted to allow a part of the laser beam, scattered by the tissue, to re-enter into the laser. The device further comprises a photo sensor for measuring light emitted from the laser, thus obtaining a signal which varies in accordance with the interference between the original laser beam and the scattered laser beam, processing means, applying a Fourier transform to the signal in order to provide its spectrum, and applying an exponential fit to the spectrum, thereby obtaining parameters corresponding to the average blood cell velocity in the tissue and the amount of blood in the tissue.

This device provides advantages corresponding to the above defined method.

Preferably, the laser is arranged to illuminate the tissue through a lens, preferably having a focal length less than 2 mm. This lens collects the scattered light, such that more light re-enters into the laser. This provides a more accurate signal.

The gap between the laser and the lens is preferably less than 2 mm.

In a preferred embodiment, the lens is accessible, such that it can be touched by a user's finger. This will make is possible to keep the finger still, such that less Doppler shift, induced by finger movement, is caused. The recorded signal will thus be more exclusively dependent on blood cell movements.

These and other aspects of the invention will be apparent from and elucidated with reference to the embodiments described hereinafter.

Fig 1 illustrates a device according to an embodiment of the invention.
Fig 2 is a flow chart, illustrating steps in a method according to an embodiment of the invention.
Fig 3 illustrates an electric signal, obtained from the photo diode of the device in fig 1.
Fig 4 illustrates the spectrum in the frequency domain of a signal such as the signal in fig 3.

Fig 5 illustrates an amplitude signal obtained from a parameter set.

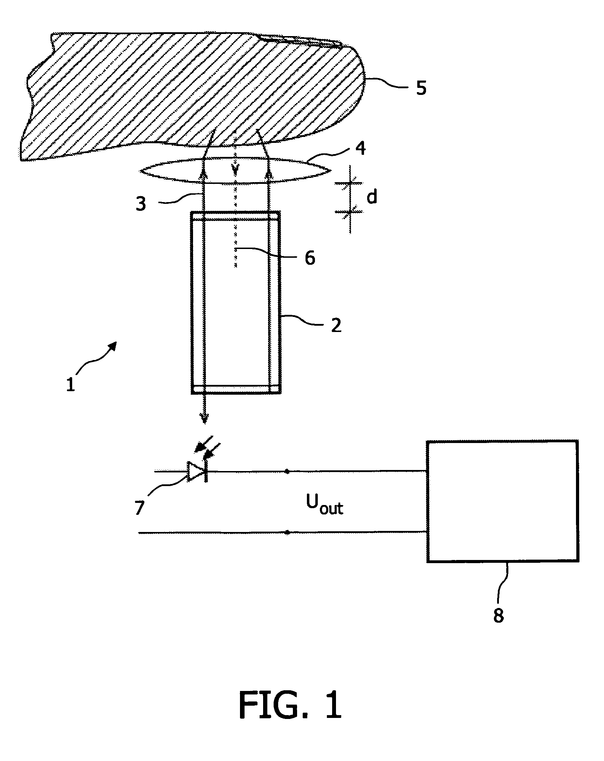

Fig 1 illustrates, schematically and in cross section, a device, a blood-flow sensor 1, according to an embodiment of the invention. The sensor 1 comprises a laser 2 in the form of a laser diode. The laser 2 may have a power of 1 mW and generates a laser beam 3 with a wavelength of 810nm.

At a close distance d from the laser a preferably convex lens 4 is placed. The laser beam 3 passes through the lens and illuminates living tissue, in this case a user's finger 5 (which should of course not be regarded as forming part of the sensor 1). The laser beam is scattered by the finger tissue, in part by red blood cells (erythrocytes) flowing in vessels in the finger 5. A part of the scattered light propagates as a reflected beam 6, collected by the lens 4, and re-enters into the laser, where it interferes with the emitted light beam 3. This means that the emitted light will comprise a speckle pattern that is depending on the Doppler velocity of the objects that scattered the laser beam 3. A part of the emitted laser light may be provided to a photo sensor 7 to obtain an electric signal corresponding to the light output, e.g. at the other side of the laser 2, as seen from the lens 4. The electric signal $U_{out}$ from the photo sensor 7 is then processed by a signal processing unit 8, as will be described later, in order to determine relevant blood-flow parameters from the signal. The signal processing unit 8 may be realized as software and/or hardware. If a high power laser is used, the lens may be omitted, since enough light may still re-enter into the laser.

The blood-flow sensor 1 is for example a self-mixing interferometer. It can be made very compact and is therefore suitable for miniaturization. It can be integrated into portable consumer products, such as a mobile phone.

A general self-mixing sensor, not intended for blood flow sensing, but resembling the above described sensor, is disclosed in WO 02/37410 A1. This sensor is used as an input device.

In an exemplary embodiment of the invention, the distance d between the laser 2 and the lens 4 is kept small, preferably 2 mm or less. This provides more light in the reflected beam 6, re-entering into the laser 3. Preferably, for the same reason the focal length of the lens 4 is kept small, preferably less than 2 mm and perhaps as small as 1 mm.

Advantageously the lens is accessible to the user such that the tissue, e.g. the finger, can be kept in contact with the lens during the measuring. This provides a better output signal, since the finger 5 cannot move relative to the sensor 1, thus introducing a Doppler shift that is unrelated to the blood flow. If for instance the flow sensor is integrated into a mobile phone, the lens may be placed in the mobile phone housing. As an alternative, the finger can be kept in contact with a transparent surface (not shown) placed on top of lens 4.

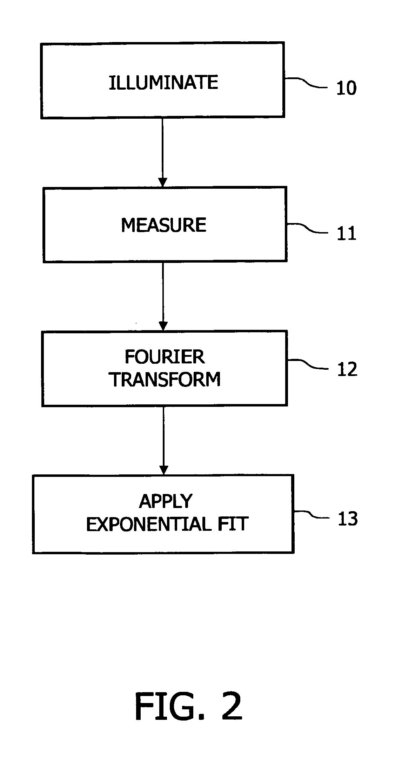

Fig 2 is a flow chart, illustrating steps in a method according to an embodiment of the invention. This method will now be described referring to figs 2-4.

An electric signal is produced using the arrangement of fig 1. Thus in a first step the tissue is illuminated 10 with a laser beam, using the laser, and a part of the laser beam, which is scattered by the target tissue is allowed to re-enter the laser. The light emitted from the laser is measured 11 with the photo sensor. A signal, which varies in accordance with the interference between the original laser beam and the scattered laser beam is thus obtained.

It can be assumed that a large number of illuminated red blood cells will reflect the laser beam in such a way that the reflected light re-enters the laser and causes a self-mixing effect. Since these blood cells move in all directions in the tissue, the resulting overall Doppler signal will not be a single sinusoidal function. Instead each individual blood cell will produce a signal with a Doppler shift that depends on that blood cells velocity in the direction of the laser beam. Higher average blood cell velocities result in laser light fluctuations with higher frequencies, lower velocities result in lower frequencies.

The Doppler shift between the outgoing and re-entering beams is the source of the speckle (fluctuation) pattern and is the sum of a large number of sinusoidal functions. The photodiode registers the speckle pattern.

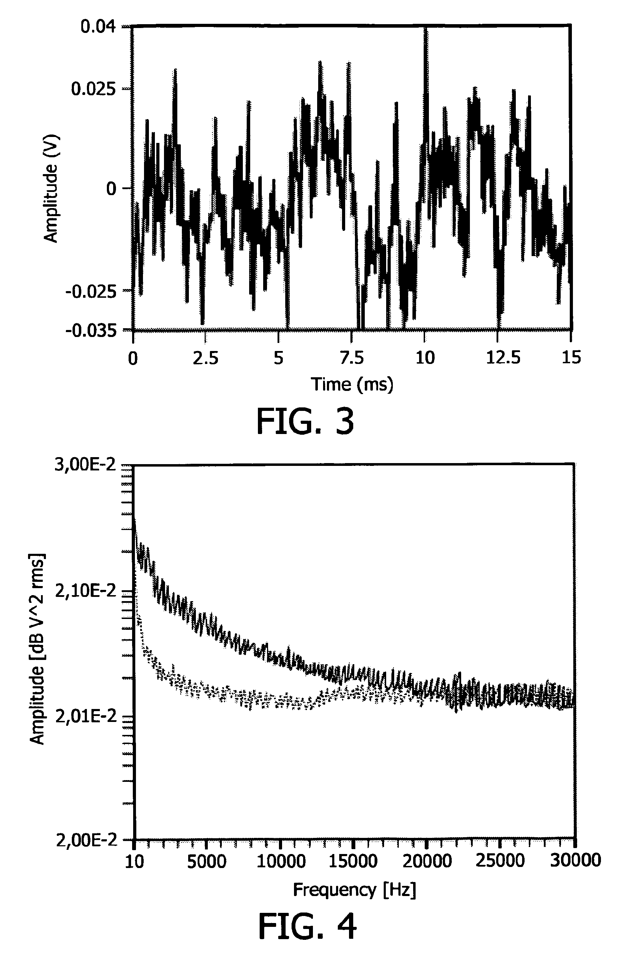

Fig 3 illustrates an example of electric signal, obtained from the photo diode of the device in fig 1. This signal appears random. From the foregoing however, it can be assumed that the spectrum of this signal will be indicative of the blood cell velocities in the tissue.

Therefore, a Fourier transform is applied to said signal in order to provide the spectrum of the electric signal.

Fig 4 shows the spectrum 20 in the frequency domain of a signal such as the signal in fig 3. A corresponding signal 21, obtained when a cuff is inflated around the user's finger in order to substantially reduce the blood flow, is also shown in fig 4. The difference between the two curves implies that the greater part of the spectral energy in the signal of fig 3 originates from moving blood cells in the user's finger.

The spectrum of such a signal can be expected to be an exponential function, see e.g. R. Bonner and R. Nossal "Model for laser Doppler measurements of blood flow in tissue" Applied Optics, Vol. 20, 2097 (1981). The spectrum can then be regarded in accordance with the following model:

$$S(t,\omega) = A(t) \cdot e^{\left(-\frac{a\sqrt{12\xi}}{\sqrt{V^2(t)}}\omega\right)}$$

where S is the spectral energy, A is the amplitude (a measure of the number of reflecting blood cells), a is the average radius of the blood cells (e.g. 0.27μm), ξ is the asymmetry of the blood cells (a measure of how light is scattered by the blood cells, ranging from 0 (light scattered uniformly in all directions) to 1 (light reflected like in a mirror), e.g. 0.1, ), and V is the average velocity of the blood cells. The inventors have found that such a model is relevant also for a signal obtained by means of a self-mixing laser sensor.

An assumption can be made that, during a short period of time, the amplitude A as well as the average velocity V will be constant. This applies e.g. for a 10 ms measuring time, since the cycle of the variation, i.e. the pulse, can be assumed to be 1 second for a resting person. Thus for the spectrum of a signal recorded during a 1 ms time slot, the amplitude A and the velocity V may be determined by performing an exponential fit of the function:

$$S = A \cdot e^{-\frac{k}{|V|}}$$

to the determined spectrum.

Thus in a fourth step an exponential fit is applied 13 to the obtained spectrum, resulting in parameters corresponding to the average blood cell velocity (V) in the tissue and the amount of blood (A) in the tissue. By applying an exponential fit is meant that an exponential function as the one indicated above is adjusted in such a way that it corresponds to the obtained spectrum. This procedure is well known per se, and is available in mathematical toolboxes, such as in LABVIEW. Through this procedure, measures of the average blood cell velocity (V) and of the amplitude (A), i.e. the amount of blood in the tissue, are obtained. The exponential fit may be applied e.g. in the interval from 0.2 to 10 kHz, which for an 810 nm laser corresponds to velocities from 0.15 mm/s to 8 mm/s. It is also possible to band pass filter the photo sensor signal in this frequency range before applying a Fourier transform.

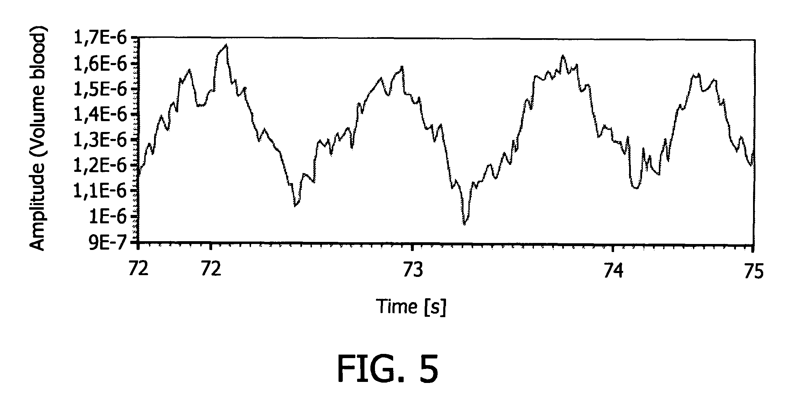

These values are particularly useful together with corresponding values, obtained for other time slots. The length of a time slot should be short enough to consider the amount of blood cells as well as the average velocity of theses cells constant. Time slots of 5-15 ms are considered suitable, but time slots as long as 50 ms can be conceivable for some applications. If 10 ms is chosen as the measuring time for determining the spectra, 100 consecutive values of the amplitude can be obtained per second. These values constitute a parameter set that can be displayed to the user as a graph, using a display, such that the variations corresponding to the pulse are clearly visible, as indicated in fig 5.

The pulse can be determined from the data by measuring the distance between the peaks in fig 5, as is well known per se. The values of the average velocities will vary in a similar way. Therefore also the velocity values may be used to determine the user's pulse.

By calculating the product of the amplitude A and the average velocity V, a measure of the perfusion P (=A*V) can be obtained. This parameter is particularly interesting, since it is depending on the first derivative of the blood pressure, which is a significant parameter for determining a user's health status.

In summary, the invention relates to a method for measuring blood flow in living tissue and a device for performing the method. The device comprises a laser and a photo sensor. The laser is arranged to illuminate the tissue in such a way that a portion of the light beam, scattered by the tissue, re-enters the laser in order to obtain a self-mixing effect. The resulting light, that is registered as an electric signal by the photo sensor, contains a speckle pattern that is depending on blood cell movement in the tissue. A Fourier transform is applied to this signal and an exponential fit is applied to the resulting frequency domain spectrum. Thereby parameters corresponding to the amount of blood cells and the average velocity of these cells may be obtained.

An application of a method and system of the invention is to implement the proposed self-mixing interferometer sensor into a programmable push-button, which reacts according to the age of the person pushing the button. It is well-known that the heart rate varies with age and that children have a higher rate beat than adults. A table may be stored in a memory coupled to the sensor that contains heart beat ranges of authorized and non-authorized persons. Thus, when a user depresses the button, his or her heart beat is measured as shown above and the measured heart beat is compared with the authorized and non-authorized ranges. The request associated with the push of the button may then be accepted or rejected depending on the pre-established rules.

In a similar fashion, a push-button may be programmed to react to human activation only. Thus, if no heart-beat is detected on the push of the button if for example the button was inadvertently depressed by mistake (clothes for example) or if the button was not pushed for a sufficient duration when depressed by mistake for example, the button will not activate any command.

The invention is not restricted to the described embodiments. It can be altered in different ways within the scope of the appended claims.

**Claims**

1. A method for measuring the blood flow in living tissue, comprising the following steps:

    - illuminating (10) the tissue with a laser beam, using a laser, and allowing a part of the laser beam, scattered by the tissue, to re-enter into the laser;
    - measuring (11), using a photo sensor, light emitted from the laser, thus obtaining a signal which varies in accordance with the interference between the original laser beam and the scattered laser beam;
- applying a Fourier transform (12) to said signal in order to provide the spectrum of the signal; **characterised by**
- applying an exponential fit (13) to said spectrum, thereby obtaining parameters corresponding to the average blood cell velocity in the tissue and the amount of blood in the tissue.

2. A method according to claim 1, wherein, for each generated spectrum, the photo sensor signal measured during a time period of 5-15 ms is used.

3. A method according to claim 2, wherein a parameter set is collected from a number of consecutive time periods.

4. A method according to claim 3, wherein a pulse is determined based on said parameter set.

5. A method according claim 1, wherein a perfusion parameter is determined by multiplying said parameter corresponding to the average blood cell velocity in the tissue and said parameter corresponding to the amount of blood in the tissue.

6. A method according to Claim 1, wherein said exponential fit is applied in an interval from 0.2 kHz to 10 kHz.

7. Device for measuring the blood flow in living tissue, comprising:

   a laser (2) for illuminating the tissue with a laser beam (3), the laser being adapted to allow a part (6) of the laser beam, scattered by the tissue, to re-enter into the laser;
   a photo sensor (7) for measuring light emitted from the laser, thus obtaining a signal which varies in accordance with the interference between the original laser beam and the scattered laser beam; **characterised in that** said device further comprises
   processing means (8) adapted to apply a Fourier transform to said signal in order to provide the spectrum of the signal, and to apply an exponential fit to said spectrum, thereby obtaining parameters corresponding to the average blood cell velocity in the tissue and the amount of blood in the tissue.

8. Device according to claim 7, wherein the laser is arranged to illuminate the tissue through a lens (4).

9. Device according to claim 8, wherein the focal length of said lens is 2 mm or less.

10. Device according to claim 8, wherein a gap between the laser and the lens is less than 2 mm.

11. Device according to claim 8, wherein the lens is accessible, such that it can be touched by a user's finger.

12. Device according to claim 7, wherein the device further comprises:

   a button for selectively generating a control command when actuated;
   and wherein, the control command is sent based on the parameters obtained by the processing means and a preset internal rule.

13. Device according to claim 12, wherein the preset internal rule includes generating the control command when a parameter representative of a human heart beat is within a preset range.

14. Device according to claim 12, wherein the preset internal rule includes generating the control command when the processing means is capable of obtaining parameters representative of a person pressing the button.


**Patentansprüche**

1. Verfahren zum Messen des Blutflusses in lebendem Gewebe, wobei das Verfahren Schritte umfasst, wonach:

   - das Gewebe unter Verwendung eines Lasers mit einem Laserstrahl beleuchtet (10) wird und ein durch das Gewebe gestreuter Teil des Laserstrahls wieder in den Laser eintreten kann;
   - von dem Laser emittiertes Licht unter Verwendung eines Fotosensors gemessen (11) wird, womit ein Signal erhalten wird, welches gemäß der Interferenz zwischen dem ursprünglichen Laserstrahl und dem gestreuten Laserstrahl variiert;
   - eine Fourier-Transformation (12) auf das Signal angewandt wird, um das Spektrum des Signals vorzusehen,

   **dadurch gekennzeichnet, dass**

   - ein Exponential Fit (13) auf das Spektrum angewandt wird, wodurch Parameter entsprechend der durchschnittlichen Blutzellengeschwindigkeit in dem Gewebe und der Blutmenge in dem Gewebe erhalten werden.

2. Verfahren nach Anspruch 1, wobei für jedes erzeugte Spektrum das während einer Zeitperiode von 5-15 ms gemessene Fotosensorsignal verwendet wird.

3. Verfahren nach Anspruch 2, wobei aus einer Anzahl von aufeinander folgenden Zeitperioden ein Parametersatz gesammelt wird.

4. Verfahren nach Anspruch 3, wobei auf der Grundlage des Parametersatzes ein Impuls ermittelt wird.

5. Verfahren nach Anspruch 1, wobei durch Multiplizieren des Parameters entsprechend der durchschnittlichen Blutzellengeschwindigkeit in dem Gewebe und des Parameters entsprechend der Blutmenge in dem Gewebe ein Perfusionsparameter ermittelt wird.

6. Verfahren nach Anspruch 1, wobei der Exponential Fit in einem Intervall von 0,2 kHz bis 10 kHz angewandt wird.

7. Vorrichtung zur Messung des Blutflusses in lebendem Gewebe, mit:

einem Laser (2) zur Beleuchtung des Gewebes mit einem Laserstrahl (3), wobei der Laser so angepasst ist, dass ein durch das Gewebe gestreuter Teil (6) des Laserstrahls wieder in den Laser eintreten kann;
einem Fotosensor (7) zum Messen von, von dem Laser emittiertem Licht, womit ein Signal erhalten wird, welches gemäß der Interferenz zwischen dem ursprünglichen Laserstrahl und dem gestreuten Laserstrahl variiert;

**dadurch gekennzeichnet, dass** die Vorrichtung weiterhin

Verarbeitungsmittel (8) umfasst, welche so eingerichtet sind, dass sie eine Fourier-Transformation auf das Signal anwenden, um das Spektrum des Signals vorzusehen, und ein Exponential Fit auf das Spektrum anwenden, wodurch Parameter entsprechend der durchschnittlichen Blutzellengeschwindigkeit in dem Gewebe und der Blutmenge in dem Gewebe erhalten werden.

8. Vorrichtung nach Anspruch 7, wobei der Laser angeordnet ist, um das Gewebe durch eine Linse (4) zu beleuchten.

9. Vorrichtung nach Anspruch 8, wobei die Brennweite der Linse 2 mm oder weniger beträgt.

10. Vorrichtung nach Anspruch 8, wobei ein Zwischenraum zwischen dem Laser und der Linse weniger als 2 mm beträgt.

11. Vorrichtung nach Anspruch 8, wobei die Linse leicht zugänglich ist, so dass sie von einem Finger des Benutzers berührt werden kann.

12. Vorrichtung nach Anspruch 7, wobei diese weiterhin umfasst:

eine Taste zur selektiven Erzeugung eines Steuerbefehls bei Aktivierung;
und wobei der Steuerbefehl auf der Grundlage der von den Verarbeitungsmitteln erhaltenen Parameter und einer vorgegebenen, internen Richtlinie übermittelt wird.

13. Vorrichtung nach Anspruch 12, wobei die vorgegebene, interne Richtlinie die Erzeugung des Steuerbefehls umfasst, wenn sich ein einen menschlichen Herzschlag darstellender Parameter innerhalb eines vorgegebenen Bereichs befindet.

14. Vorrichtung nach Anspruch 12, wobei die vorgegebene, interne Richtlinie die Erzeugung des Steuerbefehls umfasst, wenn die Verarbeitungsmittel imstande sind, Parameter zu erhalten, welche die Betätigung der Taste durch eine Person darstellen.

**Revendications**

1. Procédé pour mesurer le débit sanguin dans du tissu vivant, comprenant les étapes suivantes :

- illuminer (10) le tissu avec un faisceau laser, en utilisant un laser, et permettant à une partie du faisceau laser, diffusée par le tissu, d'entrer à nouveau dans le laser ;
- mesurer (11), en utilisant un capteur optique, la lumière émise à partir du laser, obtenant ainsi un signal qui varie en fonction de l'interférence entre le faisceau laser initial et le faisceau laser diffusé ;
- appliquer (12) une transformée de Fourier audit signal en vue de fournir le spectre du signal ;
**caractérisé par**
- appliquer (13) un ajustement exponentiel audit spectre, obtenant ainsi des paramètres correspondant à la célérité moyenne des globules sanguins dans le tissu et la quantité de sang dans le tissu.

2. Procédé selon la revendication 1, dans lequel, pour chaque spectre généré, le signal du capteur optique mesuré durant une période de 5 à 15 ms est utilisé.

3. Procédé selon la revendication 2, dans lequel un ensemble de paramètres est collectés à partir d'un nombre de périodes de temps consécutives.

**4.** Procédé selon la revendication 3, dans lequel une impulsion est déterminée sur la base dudit ensemble de paramètres.

**5.** Procédé selon la revendication 1, dans lequel un paramètre de perfusion est déterminé en multipliant ledit paramètre correspondant à la célérité moyenne des globules sanguins dans le tissu et ledit paramètre correspondant à la quantité de sang dans le tissu.

**6.** Procédé selon la revendication 1, dans lequel l'ajustement exponentiel est appliqué dans un intervalle de 0,2 kHz à 10 kHz.

**7.** Dispositif pour mesurer le débit sanguin dans du tissu vivant, comprenant :

- un laser (2) pour illuminer le tissu avec un faisceau laser (3), le laser étant adapté pour permettre à une partie (6) du faisceau laser, diffusée par le tissu, d'entrer à nouveau dans le laser ;
- un capteur optique (7) pour mesurer la lumière émise à partir du laser, obtenant ainsi un signal qui varie en fonction de l'interférence entre le faisceau laser initial et le faisceau laser diffusé ; **caractérisé en ce que** ledit dispositif comprend en outre :
- des moyens de traitement (8) adaptés pour appliquer une transformée de Fourier audit signal en vue de fournir le spectre du signal, et d'appliquer un ajustement exponentiel audit spectre, obtenant ainsi des paramètres correspondant à la célérité moyenne des globules sanguins dans le tissu et la quantité de sang dans le tissu.

**8.** Dispositif selon la revendication 7, dans lequel le laser est disposé pour illuminer le tissu à travers une lentille (4).

**9.** Dispositif selon la revendication 8, dans lequel la distance focale de ladite lentille est de 2 mm ou moins.

**10.** Dispositif selon la revendication 8, dans lequel l'écartement entre le laser et la lentille est inférieur à 2 mm.

**11.** Dispositif selon la revendication 8, dans lequel la lentille est accessible, de sorte qu'elle puisse être touchée par le doigt d'un utilisateur.

**12.** Dispositif selon la revendication 7, dans lequel le dispositif comprend en outre :

un bouton pour générer de façon sélective une commande de contrôle lorsqu'il est actionné ; et dans lequel, la commande de contrôle est envoyée sur la base des paramètres obtenus par les moyens de traitement et une règle interne préréglée.

**13.** Dispositif selon la revendication 12, dans lequel la règle interne préréglée inclut la génération de la commande de contrôle lorsqu'un paramètre représentatif du battement de coeur humain est dans un intervalle préréglé.

**14.** Dispositif selon la revendication 12, dans lequel la règle interne préréglée inclut la génération de la commande de contrôle lorsque les moyens de traitement sont capable d'obtenir des paramètres représentatifs d'une personne pressant le bouton.

FIG. 1

```
┌─────────────────────┐
│     ILLUMINATE      │ ── 10
└─────────────────────┘
          │
          ▼
┌─────────────────────┐
│      MEASURE        │ ── 11
└─────────────────────┘
          │
          ▼
┌─────────────────────┐
│      FOURIER        │
│    TRANSFORM        │ ── 12
└─────────────────────┘
          │
          ▼
┌─────────────────────┐
│       APPLY         │
│  EXPONENTIAL FIT    │ ── 13
└─────────────────────┘
```

# FIG. 2

FIG. 3

FIG. 4

FIG. 5

EP 1 858 404 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

*   EP 282210 A1

*   WO 0237410 A1

**Non-patent literature cited in the description**

*   Non invasive blood flow measurement using speckle signals from a self-mixing laser diode: in vitro and in vivo experiments. *Optical Engineering SPIE USA,* September 2000, vol. 39 (9), 2574-2580
*   Self-mixing in a diode laser as a method for cardiovascular diagnostics. *Journal of Biomedical Optics SPIE USA,* January 2003, vol. 8 (1), 152-160

*   Simple coherence method for blood flow detection. *Proceedings of the SPIE - The International Society for Optical Engineering,* 2000, vol. 3915, 112-120
*   **R. Bonner ; R. Nossal.** Model for laser Doppler measurements of blood flow in tissue. *Applied Optics,* 1981, vol. 20, 2097